Europäisches Patentamt

European Patent Office

Office européen des brevets

(11) Publication number: **0 221 378**
**B1**

# EUROPEAN PATENT SPECIFICATION

(45) Date of publication of patent specification: **03.10.90**

(21) Application number: **86113858.4**

(22) Date of filing: **25.02.85**

(60) Publication number of the earlier application in accordance with Art. 76 EPC: **0 156 517**

(51) Int. Cl.⁵: **B 65 F 1/16**

(54) Containers.

(30) Priority: **01.03.84 GB 8405390**

(43) Date of publication of application:
**13.05.87 Bulletin 87/20**

(45) Publication of the grant of the patent:
**03.10.90 Bulletin 90/40**

(84) Designated Contracting States:
**CH DE FR GB IT LI NL SE**

(56) References cited:
**FR-A-2 505 639**
**GB-A-2 087 360**
**US-A-1 360 732**
**US-A-1 984 228**

(73) Proprietor: **Frontier Plastics (South Wales) Ltd.**
**North Blackvein Industrial Estate**
**Crosskeys Gwent (GB)**

(72) Inventor: **Harris, John**
**"Trevorrick" 144, Risca Road**
**Newport Gwent Wales (GB)**
Inventor: **Anthony, John Edgar**
**Little Paddocks Fields Park Road**
**Newport Gwent Wales (GB)**

(74) Representative: **Wynne-Jones, John Vaughan et al**
**Wynne-Jones, Lainé & James Morgan Arcade Chambers 33 St. Mary Street**
**Cardiff CF1 2AB (GB)**

Courier Press, Leamington Spa, England.

## Description

This invention relates to containers for refuse or toxic waste or disposables, and is particularly, through not exclusively, applicable to containers for medical refuse or the like which may include toxic or contaminated materials sometimes in conjunction with broken glass or sharp objects such as used hypodermic syringes. Disposal of these classes of material presents serious problems and the object of the invention is to provide an improved container for this general purpose.

One of the requirements is that it should be possible to insert the refuse into the container without the operator's hand coming into contact with contaminated material or surfaces. The entry to the container should, however, be closable to prevent material accidentally escaping and to prevent insects or other small creatures from entering or leaving. The container should also be capable of accepting comparatively large elongated objects such as long length hypodermic syringes, and in any case when full it should be possible to close and seal the container to prevent unintentional or unauthorised opening before it is disposed of, for example by burial or autoclave, or in an incinerator. Another object of the invention is to provide an improved closure system which in normal use will allow objects to be introduced freely, but will automatically close in a non-positive manner, and which can, when required, be closed positively and irreversibly. It is also important that any part of the closure which may come into contact with contaminated materials should not be contacted by hand or exposed externally when the container is sent for disposal.

The invention relates to the type of container (suitable for refuse or toxic waste or disposables), having an upper entry chute, a closure arranged to selectively open and close the lower inner end of the chute and means for closing the outer end of the chute in the form of a door connected to the container by a hinge. (known from US—A—1360 732).

In accordance with the invention the hinge comprises a flexible element which prevents egress of the contents through the flexible hinge, the door is provided with a locking detent arranged to engage the body of the container when the door is closed and which acts as a non-reversible locking element.

The container will normally be manufactured in at least three separate parts, a top, bottom and closure, and the design of the connections between these parts is important. Preferably there is a snap-fitting irreversible connection between the top and bottom, the connection also being arranged to prevent relative inward movement of the side walls of either part in relation to the other.

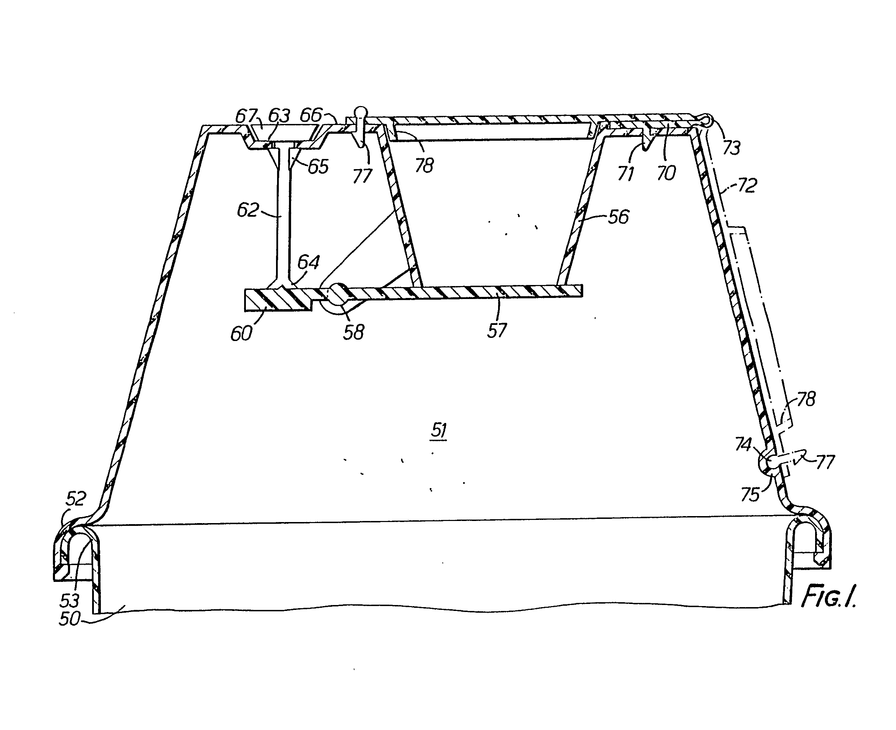

The invention may be performed in various ways and one specific embodiment will now be described by way of example with reference to the accompanying drawing, which is a diagrammatic sectional side elevation through an example of the invention.

The container for disposable items comprises a main base part 50 and an upper part 51 which are positively and irreversibly engaged by means of a down-turned rim 52 on the upper part gripping over the out-turned rim 53 on the lower part. The upper part 51 is formed with an entry chute 56 and at the lower end of this there is provided a pivoted door or gate 57 mounted on a pivot 58 within the container and having a counterweight 60 which tends to hold the door close against the lower end of the chute. To introduce an object into the container it is sufficient merely to drop it into the chute and the door opens automatically and closes by gravity. When the container is full the door can be locked positively closed by means of a locking plug 62 which is inserted through an opening 63 in the upper wall and has an inverted notch 64 at its lower end to engage over a rib on the counterweight 60. The plug also has a number of irreversible barbs or detents 65 which engage below the top wall 66 and prevent the plug being withdrawn. A knob or disc 67 at the top of the plug fits into a recess and prevents material escaping through the aperture.

The sloping surfaces of the chute 56 may become contaminated during use and the container includes means to close off the outer or upper end of the chute when the container is full. A secondary closure is attached to the upper part, comprising an element (static leaf) 70 having a number of barbed locking formations 71, which engage with small apertures in the top of the container, and a hinged leaf 72 connected to the static leaf by a thin flexible strip or "live hinge" 73. During normal use the flap 72 lies in the inclined position shown in chain lines and is held in that positive by a pip or bead 74 engaging in a socket 75 in the container wall. When the container is full the flap 72 is swung upwards and over into a horizontal position in which a second barbed detent 77 engages in a small hole in the top wall 66, and a shallow rim or flange 78 makes a tight snap fit in the upper end of the chute. This effectively closes both the inner and outer ends of the chute.

## Claims

1. A container for refuse or toxic waste or disposables, having an upper entry chute (56), a closure (57) arranged to selectively open and close the lower inner end of the chute and means for closing the outer end of the chute in the form of a door connected to the container by a hinge, characterised in that the hinge comprises a flexible element (73) which prevents egress of the contents through the flexible hinge, the door (72) is provided with a locking detent (77) arranged to engage the body of the container (50, 51) when the door is closed and which acts as a non-reversible locking element.

2. A container according to claim 1, in which the locking detent (77) is barbed.

3. A container according to claim 1 or claim 2 in which the door (72) has a detent (74) to engage part of the body of the container (50, 51) to hold the door (72) in an open position away from the mouth of the chute.

4. A container according to any of the preceding claims, in which the door (72) has a projecting rim (78) on its underside arranged to fit closely into the mouth of the chute (56).

5. A container according to any of the preceding claims, in which the door (72) is hinged to an element (70) which is removably attached to the body of the container by a locking formation (71).

6. A container according to any of the preceding claims, in which the closure (57) at the inner end of the chute (56) is arranged to be locked in its closed position by means of a locking element (62).

7. A container according to any of the preceding claims in which the hinge for the door (72) is integrally moulded with the door.

## Patentansprüche

1. Behälter für Müll oder giftigen Abfall oder Einwegartikel, mit einer oberen Einfüllschütte (56), einem Deckel (57) zum wahlweisen Freigeben oder Verschließen des unteren inneren Endes der Schütte, und Mitteln in Form einer durch ein Scharnier am Behälter befestigten Tür zum Verschließen des äußeren Endes der Schütte, dadurch gekennzeichnet, daß das Scharnier ein flexibles Element (73) aufweist, das ein Austreten des Inhalts durch das biegsame Scharnier verhindert, und daß die Tür (72) mit einer Rastvorrichtung (77) versehen ist, welche mit dem Behälter (50, 51) zusammenwirkt, wenn die Tür geschlossen ist und welche als irreversibles Sperrelement wirkt.

2. Behälter nach Anspruch 1, wobei die Sperrvorrichtung (77) einen Widerhaken aufweist.

3. Behälter nach Anspruch 1 oder 2, wobei die Tür (72) eine Raste (74) aufweist, die mit einem Teil des Behälters (50, 51) zusammenwirkt, um die Tür (72) in einer offenen Stellung weg von der Mündung der Schütte zu halten.

4. Behälter nach einem der vorhergehenden Ansprüche, wobei die Tür (72) an ihrer Unterseite einen hochstehenden Rand (78) aufweist, der eng in die Mündung der Schütte paßt.

5. Behälter nach einem der vorhergehenden Ansprüche, wobei die Tür (72) scharnierartig an einem Element (70) befestigt ist, das durch ein Sperrglied (71) lösbar mit dem Behälter verbunden ist.

6. Behälter nach einem der vorhergehenden Ansprüche, wobei der Deckel (57) am inneren Ende der Schütte (56) mittels eines Verriegelungselements (62) in seiner Schließstellung gesperrt ist.

7. Behälter nach einem der vorhergehenden Ansprüche, wobei das Scharnier für die Tür (72) einstückig mit der Tür geformt ist.

## Revendications

1. Conteneur pour détritus ou déchets toxiques, ayant une chute (56) présentant une entrée supérieure, une fermeture (57) placée de façon à ouvrir ou fermer sélectivement la partie inférieure de la chute et des moyens pour fermer l'extrémité extérieure de la chute sous forme d'une porte reliée au conteneur par une charnière, caractérisé en ce que la charnière comprend un élément flexible (73), qui empêche la sortie du contenu à travers la charnière flexible, que la porte (72) est munie d'un cliquet de verrouillage (77) placé pour s'engager avec le corps du conteneur (55, 51) quand la porte est fermée et qui agit comme un élément de verrouillage non-réversible.

2. Conteneur selon la revendication 2, dans lequel le cliquet (77) de verrouillage a un ardillon.

3. Conteneur selon la revendication 1 ou la revendication 2, dans lequel la porte (72) présente un cliquet (74) pour s'engager avec une partie du corps du conteneur (50, 51) afin de maintenir la porte (72) en position ouverte dégagée par rapport à l'embouchure de la chute.

4. Conteneur selon l'une des revendications précédentes, dans lequel la porte (72) présente une bordure (78) faisant saillie sur sa face inférieure, placée de façon à s'adapter étroitement avec l'embouchure de la chute (56).

5. Conteneur selon l'une des revendications précédentes, dans lequel la porte (72) est fixée par une charnière à un élément (70) qui est attaché, mais détachable du corps du conteneur, au moyen d'un dispositif de verrouillage (71).

6. Conteneur selon l'une des revendications précédentes, dans lequel la fermeture (50) à l'extrémité intérieure de la chute (56) est placée pour être verrouillée en position fermée au moyen de l'élément de verrouillage (62).

7. Conteneur selon l'une des revendications précédentes, dans lequel la charnière de la porte (72) est moulée de façon intégrante avec la porte.

Fig.1.